(19) Europäisches Patentamt
European Patent Office
Office européen des brevets

(11) Veröffentlichungsnummer: **0 230 397 B1**

(12) **EUROPÄISCHE PATENTSCHRIFT**

(45) Veröffentlichungstag der Patentschrift: **14.04.93**

(51) Int. Cl.$^5$: **C07H 15/04**, A01N 43/16

(21) Anmeldenummer: **87810015.5**

(22) Anmeldetag: **12.01.87**

(54) **Neues Glucopyranosid.**

(30) Priorität: **17.01.86 CH 192/86**

(43) Veröffentlichungstag der Anmeldung:
**29.07.87 Patentblatt 87/31**

(45) Bekanntmachung des Hinweises auf die Patenterteilung:
**14.04.93 Patentblatt 93/15**

(84) Benannte Vertragsstaaten:
**AT CH DE FR IT LI**

(56) Entgegenhaltungen:

**Z. ANGEW. ENTOMOL, Band 80, 1976, Seiten 50-56, Verlag Paul Parey, Hamburg, Berlin, DE; J. HURTER et al.: "Beitrag zur Anreicherung und teilweisen Reinigung des eiablageverhindernden Pheromons der Kirschenfliege, Rhagoletis cerasi L. (Dipt., Trypetidae)**

**EXPERIENTIA, Band 43, No. 2, Februar 1987, Seiten 157-164, Birkhäuser Verlag, Basel, CH; J. HURTER et al.: "Oviposition-deterring pheromone in Rhagoletis cerasi L.: Purification and determination of the chemical constitution"**

(73) Patentinhaber: **Eidgenössische Forschungsanstalt für Obst-, Wein- und Gartenbau**

**CH-8820 Wädenswil(CH)**

(72) Erfinder: **Boller, Ernst, Dr.**
**Büelenebnetstrasse 27**
**CH-8820 Wädenswil(CH)**
Erfinder: **Hurter, Jakob, Dr.**
**Hintere Bergstrasse 18**
**CH-8942 Oberrieden(CH)**
Erfinder: **Städler, Erich, Dr.**
**Palmisacker**
**CH-8821 Schönenberg(CH)**
Erfinder: **Raschdorf, Fritz**
**Kleinriehenstrasse 101**
**CH-4058 Basel(CH)**

(74) Vertreter: **Roth, Bernhard M. et al**
**Patentabteilung der CIBA-Geigy AG Klybeckstrasse 141**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 230 397 B1

Rank Xerox (UK) Business Services
(3.10/3.5x/3.0.1)

## Beschreibung

Die vorliegende Erfindung betrifft ein neues Palmityl-glucopyranosid, ein Verfahren zu seiner Gewinnung, sowie seine Verwendung Zum Schützen von Kirschen vor dem Befall durch Kirschenfliegen.

Das erfindungsgemässe Palmityl-glucopyranosid entspricht der Formel I

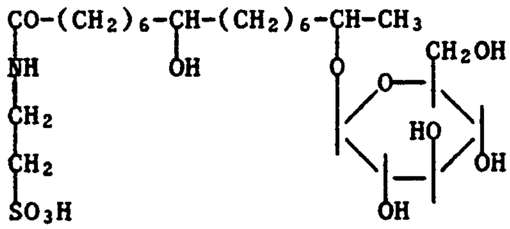

(I)

und ist somit ein N[15-(β-Glucopyranosyl)oxy-8-hydroxypalmitoyl]-taurin. In der Formel I sind alle 4 möglichen optischen Isomeren, die durch die beiden Chiralitätszentren bedingt sind, eingeschlossen. Die Verbindung der Formel I kann auch in Form ihres Natrium- oder Kalium-Salzes vorliegen.

Die Weibchen der Kirschenfliege Rhagoletis cerasi legen ihre Eier in halbreife Kirschen. Bis zum Zeitpunkt des Pflückens der reifen Früchte entwickeln sich die Larven in den Kirschen und machen diese als Tafelobst unbrauchbar. Es war somit ein dringendes Bedürfnis, ein Mittel zu finden, mit dem die heranreifenden Früchte vor der Eiablage behandelt werden können, um so die Kirschenfliege an der Eiablage zu hindern.

Auf Grund der Beobachtung, dass in der Regel in einer Kirsche nur eine einzige Larve zu finden ist, wurde die Oberfläche von befallenen Kirschen gereinigt. Der Reinigungsextrakt wurde auf heranwachsende Kirschen appliziert und eine Abnahme des Befalls durch Kirschenfliegen festgestellt. Es ist bereits aus Z. ANGEW. ENTOMOL., Band 80, 1976, Seiten 50-56 bekannt, dass der eiablagehindernde Extraktanteil an Cellulosedünnschichten und an DOWEX 2-Ionentauscherharzen angereichert werden kann.

Zur Gewinnung grösserer Mengen des Markierungsstoffes der Kirschenfliegen wurden Zuchtkäfige aus Plexiglas verwendet. Nach 2 bis 4 Wochen nach Versuchsbeginn wurden die Exkremente der Fliegen von den Glaswänden abgestossen und mit Methanol extrahiert. Die braune Lösung wurde eingeengt und mit Aethanol verdünnt. Dieser Extrakt ist bei -20 °C beliebig lange haltbar und wurde für die weitere Aufarbeitung verwendet.

Die Reinigung des Extraktes erfolgte mittels mehrfacher, abwechslungsweiser Säulen- und Dünnschicht-Chromatographie. Als Laufmittel wurden Mischungen von Methanol, Aethanol, Acetonitril und Wasser sowie die reinen Lösungsmittel verwendet.

Die Aktivität der einzelnen Fraktionen wurde im Verhaltensexperiment und elektrophysiologisch an der Kirschenfliege geprüft. Nach sechs Reinigungsoperationen war der Extrakt so rein, dass weder die FAB-Spektren (Fast-Atom-Bombardment, Beschuss der zu untersuchenden Substanz mit schnellen Atomen) noch die FD-Spektren (Feldionen Desorption, Verflüchtigung der zu untersuchenden Substanz unter dem Einfluss eines elektrischen Feldes) auf wesentliche Verunreinigungen hinwiesen.

Die eigentliche Strukturaufklärung erfolgte mit einem handelsüblichen HF-Massenspektrometer (Hoch frequenz-Massenspektrometer und einem ebenfalls handelsüblichen NMR-Spektrometer, wobei nach üblichen Methoden vorgegangen wurde. Die erhaltenen Signale wurden nach bekannten Methoden ausgewertet und bestätigten die Struktur der Formel I.

Beispiel

A. Gewinnung des Rohmaterials

Zur Gewinnung des Rohmaterials werden Zuchtkäfige aus Plexiglas (30x30x40 cm) verwendet, die mit Glasplatten ausgekleidet und zur Vergrösserung der Oberflächen mit weiteren Glasplatten senkrecht unterteilt sind. Diese Käfige werden mit einer Wasserquelle und Futter bestehend aus Hefehydrolysat und Zucker versehen. Die Kirschenfliegen (Rhagoletis cerasi) werden alsdann in diesen Käfigen zum Schlüpfen gebracht. Die auf den Glasplatten deponierten Exkremente der Kirschenfliegen werden nach 3 Wochen mit einer Rasierklinge abgestossen.

7,3 g dieser braunen, klebrigen Masse werden in 75 ml Methanol während 10 Minuten in einem Ultraschallbad aufgeschlämmt. Anschliessend wird bei 2 °C während 4 Stunden mit einem Magnetrührer

gerührt und 20 Minuten lang bei 12 000 g zentrifugiert. Der Bodensatz wird zwei weitere Male in 40 ml Methanol aufgeschlämmt und wie beschrieben extrahiert und zentrifugiert. Die vereinigten methanolischen Extrakte werden am Rotationsverdampfer auf 20 ml eingeengt und dann mit 20 ml Aethanol verdünnt (= 40 ml Rohextrakt).

B. Reinigung des Rohextraktes

1. Flüssigkeits-Chromatographie an Cellulosesäule

Ein Glasrohr 50/600 mm wird nach dem Schüttverfahren mit Cellulosepulver (123 A, Schleicher-Schüll, Dassel, BRD) in Methanol/Aethanol 1:1 auf eine Säulenhöhe von 400 mm gefüllt. 40 ml des unter (A) erhaltenen Rohextraktes werden auf diese Säule gegeben und bei einem Säulenfluss von 10-15 $ml \cdot min^{-1}$ zuerst mit 1400 ml Methanol/Aethanol 1:1 und dann mit 1000 ml Methanol/Wasser 1:1 eluiert.

Elektrophysiologisch wird im Intervall zwischen 1100 ml und 2000 ml Elutionsvolumen Aktivität nachgewiesen. Diese 900 ml werden am Rotationsverdampfer auf 0,5 ml eingeengt und in 3,5 ml Methanol aufgenommen.

2. Dünnschicht-Chromatographie an präparativer Celluloseplatte

8 PSC-Celluloseplatten mit den Massen 200/200 mm und 0,5 mm Schichtdicke, ohne Fluoreszenzindikator (Merck, Darmstadt, BRD), werden zur Reinigung einmal mit Wasser und nach Trockenwerden einmal mit Methanol bis zum oberen Rande entwickelt. In trockenem Zustand werden auf jede Platte 0,5 ml des unter B.1 erhaltenen methanolischen Konzentrates mit Hilfe eines Auftraggerätes aufgebracht. Die Entwicklung erfolgt mit Aethanol/Wasser 1:1. Nach beendeter Entwicklung wird das Schichtmaterial in Streifen von 0,1 $R_f$-Einheiten entfernt und wie folgt eluiert: Die Streifen werden in 15 ml Methanol aufgeschlämmt, 15 Minuten lang mit dem Magnetrührer extrahiert und anschliessend während 15 Minuten bei 3000 g zentrifugiert. Der Bodensatz wird nochmals mit 10 ml Methanol der gleichen Extraktion unterworfen. Die vereinigten methanolischen Lösungen werden am Rotationsverdampfer auf ein Volumen von 0,5 ml eingeengt. Elektrophysiologische Aktivität zeigt sich bei 0,9 und 1,0 $R_f$-Einheiten

3. Flüssigkeits-Chromatographie an C-18 Säule

| | |
|---|---|
| Säulentyp : | Ultrasphere-ODS (Altex, USA) 4,6x250 mm; Korngrösse 5 $\mu$. |
| Laufmittel: | 20 % Methanol in Wasser bis 100 % Methanol bei einem Gradienten von 5 % $Methanol \cdot min^{-1}$ (steiler Gradient) |
| Säulenfluss: | 0,5 $ml \cdot min^{-1}$ |
| Fraktionsvolumen: | 0,5 ml |

Der unter B.2 erhaltene Extrakt wird in Portionen von 0,1 ml auf die Säule gegeben.

Elektrophysiologisch wird im Intervall zwischen 7,5 ml und 9 ml Elutionsvolumen Aktivität nachgewiesen. Die vereinigten aktiven Fraktionen werden am Vakuum auf 0,1 ml eingeengt und dann mit Methanol auf 0,5 ml verdünnt.

4. Dünnschicht-Umkehr-Phasen-Chromatographie an C-12 Platten

Plattentyp: OPTI-UP C-12; 10x20 cm; Schichtdicke 0,25 mm mit Fluoreszenzindikator (Antec, Beinwil, CH)

5 Platten werden wie unter B.2 beschrieben gereinigt. Auf die gereinigten trockenen Platten wird je 0,1 ml des unter B.3 gewonnenen Extraktes mit einem Auftraggerät aufgebracht. Die Entwicklung der Platten erfolgt mit Methanol als Laufmittel. Nach beendeter Entwicklung wird das Schichtmaterial in Streifen von 0,1 $R_f$-Einheiten entfernt und wie folgt eluiert: Die Streifen werden je in 3 ml Methanol aufgeschlämmt und 15 Minuten lang mit einem Magnetrührer extrahiert und anschliessend während 20 Minuten bei 3000 g zentrifugiert. Die Bodensätze werden nochmals der gleichen Extraktion unterworfen und die entsprechenden Ueberstände vereinigt. Diese 10 Fraktionen werden einzeln am Rotationsverdampfer zur Trockne eingeengt und je in 0,3 ml Methanol/Acetonitril 1:1 aufgenommen.

Elektrophysiologisch wird im $R_f$-Einheitenbereich 0,5-0,7 Aktivität nachgewiesen.

5. Flüssigkeits-Chromatographie an $NH_2$-Säule

| | |
|---|---|
| Säulentyp : | Lichrosorb $NH_2$ (Merck, Darmstadt, BRD) 4x250 mm; Korngrösse 5 µ. |
| Laufmittel: | 100 % Acetonitril bis 100 % Wasser bei einem Gradienten von 6 % Wasser$\cdot$min$^{-1}$ |
| Säulenfluss: | 0,5 ml$\cdot$min$^{-1}$ |
| Fraktionsvolumen: | 0,5 ml |

Die unter B.4 erhaltenen aktiven Extrakte werden in Portionen von 0,1 ml auf die Säule gegeben.

Elektrophysiologisch wird im Intervall von 12,5 ml bis 15 ml Elutionsvolumen Aktivität nachgewiesen. In diesem Bereich wird das Wasser durch Lyophilisation entfernt und der Rückstand in 0,3 ml 40%igem Methanol/Wasser-Gemisch aufgenommen.

6. Flüssigkeits-Chromatographie an C-18 Umkehr-Phasen-Säule

| | |
|---|---|
| Säulentyp : | Ultrasphere-ODS (Altex, USA) 4,6x250 mm; Korngrösse 5 µ. |
| Laufmittel: | 40 % Methanol in Wasser bis 100 % Methanol bei einem Gradienten von 2 % Methanol$\cdot$min$^{-1}$ (flacher Gradient) |
| Säulenfluss: | 0,5 ml$\cdot$min$^{-1}$ |
| Fraktionsvolumen: | 0,5 ml |

Der unter B.5 erhaltene Extrakt wird in zwei Portionen von 0,15 ml auf die Säule gegeben. Jede der erhaltenen 50 Fraktionen wird am Rotationsverdampfer auf 0,1 ml und in einem Gefäss mit konischem Boden in einem leichten Stickstoffstrom zur Trockne eingeengt.

Die Fraktionen Nr. 16 bis Nr. 22 zeigen biologische Aktivität. Der trockene Rückstand dieser Fraktionen bildet an der Gefässwand einen farblosen, lackartigen und nicht verdampfbaren Film von hoher Polarität und ohne Lichtabsorption im UV-Bereich.

Jede der sieben aktiven Fraktionen wird in 50 µl Methanol aufgenommen. Die Fraktion Nr. 19 weist die höchste Konzentration auf und ist elektrophysiologisch bis zu einer $10^7$ fachen Verdünnung nachzuweisen. Im labormässigen Verhaltensexperiment mit Kirschenfliegen an behandelten Ceresin-Wachsdomen (E. Boller und J. Hurter: Entomol.exp.appl. 39, 163-169, 1985) wird der elektrophysiologische Befund bestätigt.

C. Elektrophysiologische Prüfung

Die Prüfung auf biologische Aktivität der Fraktionen erfolgt mittels elektrophysiologischen Ableitungen von tarsalen Kontakt-Chemorezeptoren, wobei nach der von E. Städler beschriebenen Methode vorgegangen wird (E. Städler: Contract Chemoreception; Chemical Ecology of Insects, 1984, p. 3-35). Die zu messenden Proben bestehen aus 10 µl Fraktionslösung aufgelöst in 1 ml 10 mN Natriumchlorid-Lösung. 2 µl dieser verdünnten Lösung werden in eine Glas-Kapillarelektrode aufgesogen und zur Stimulierung von 4 bis 8 Rezeptoren des Prothorax tarsus einer weiblichen Kirschenfliege verwendet.

D. Strukturaufklärung

Fast Atom Bomberdment (FAB) einer Probe von B.6 liefert ein Massenspektrum, das intensive Signale für das protonierte Molekül $[M + H]^+$ (m/z 558) sowie von Addukten des Moleküls mit Alkalimetallionen, z.B. $[M + Na]^+$ (m/z 580) und $[M + K]^+$ (m/z 596), zeigt. Als Matrix wird Thioglycerin verwendet. Eine Massenbestimmung unter Hochauflösungsbedingungen (Matrix:Polyäthylenglykol) ergibt für das $[M + Na]^+$ Ion 580,2778, was mit der Bruttoformel $C_{24}H_{47}O_{11}NSNa$ (berechnete Masse 580,2764) gut übereinstimmt.

Das 400 MHz $^1$H-NMR-Spektrum in $CD_3OD$ zeigt das Vorhandensein einer Glucose an, die $\beta$-glykosidisch mit dem $\omega$-1 Kohlenstoff eines geradkettigen Dihydroxyfettsäure-Derivates verknüpft ist. Die GC/MS-Analyse der mit Bis-trimethylsilyl-acetamid silylierten Methanolyseprodukte liefert die Information über die Kettenlänge der Dihydroxyfettsäure und die Position der freien OH-Gruppen in der Kette. Das EI-MS-Spektrum (elektische Ionisations-Massenspektroskopic) des Trimethylsilyl (TMS)-Derivates des Methylesters (M = 446, extrapoliert vom $[M\text{-}CH_3]^+$-Fragment m/z = 431 und von $[M + H]^+$-Ion m/z = 447, das durch chemische Ionisation mit Isobutan als Reaktandgas erhalten wird) zerfällt in charakteristischen $\alpha$-Brüchen in die Fragmente $TMS\text{-}\overset{+}{O}=CH(CH_2)_6COOCH_3$ m/z = 245; $CH_3CH(O\text{-}TMs)\text{-}(CH_2)_6\text{-}CH=\overset{+}{O}TMS$, m/z = 303; und $CH_3CH=O\text{-}TMS$, m/z = 117. Aus diesen Fragmenten folgen die Hydroxylpositionen C-8 und C-15 und somit auch die Kettenabschnitte m = n = 6.

Bei der Methanolyse wird als weiteres Spaltprodukt von B.6 Taurin mit Hilfe der Dünnschichtchromatographie nachgewiesen (DC-Fertigplatten, Kieselgel 60 F 254; Laufmittel n-Butanol/99%ige

Essigsäure/Wasser 8:3:1 und n-Propanol/25%iger Ammoniak 8:2; Nachweisreagenz:$Cl_2$/KJ/N,N,N',N'-Tetramethyl-4,4'-diaminodiphenylmethan).

Die auf Grund der NMR-Signale postulierte Amid-Verknüpfung zwischen Fettsäure und Taurin steht mit der Nichtacetylierbarkeit des N-Atoms von B.6 in vollem Einklang.

<u>Tabelle I:</u> Methanolyse von B.6

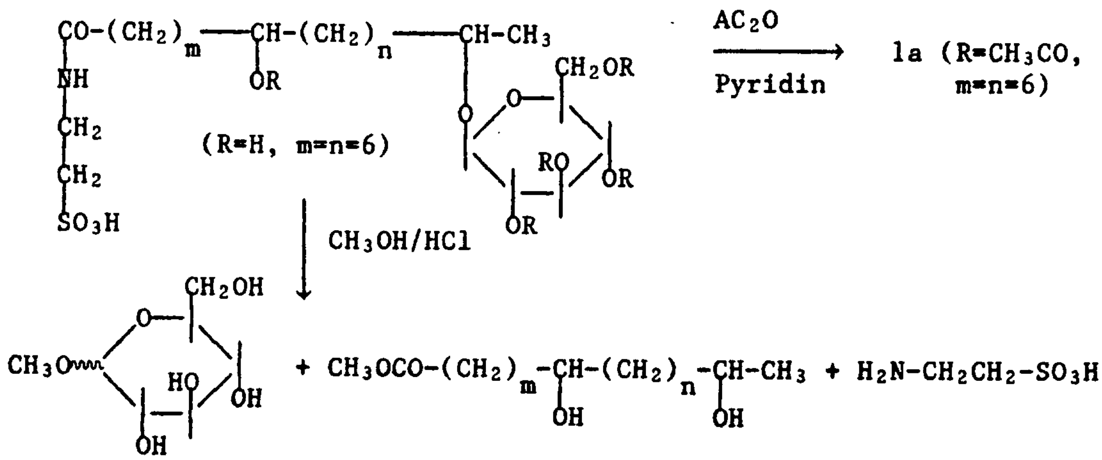

Tabelle II: 400 MHz $^1$H-NMR-Parameter von 1 und 1a in $CD_3OD$.

| H[a.)] | δ (1) | δ (1a)[b.)] | Kopplungskonstanten | | |
|---|---|---|---|---|---|
| | | | J(Hz) | 1 | 1a |
| 1 | 4.31 | 4.70 | J(1.2) | 8.0 | 8.0 |
| 2 | 3.13 | 4.84 | J(2.3) | 9.0 | 9.5 |
| 3 | 3.33 | 5.24 | J(3.4) | 9.0 | 9.5 |
| 4 | 3.28 | 4.99 | J(4.5) | 9.0 | 10.0 |
| 5 | 3.23 | 3.84 | J(5.6) | 6.0 | 2.5 |
| 6 | 3.66 | 4.11 | J(5.6) | 2.0 | 5.0 |
| 6' | 3.84 | 4.25 | J(6.6) | 12.0 | 12.5 |
| X-$CH_2$ | 2.95 | 2.94 | zwei Tripletts mit J($CH_2$, $CH_2$) = 7 Hz | | |
| Y-$CH_2$ | 3.58 | 3.58 | | | |
| Z-CO | | | | | |
| C-2 $CH_2$ | 2.18 | 2.18 | Triplett J = 7 Hz | | |
| $(CH_2)_n$ | c.) 3.50 | c.) 4.84 | c.) Multiplett | | |
| "C-i" CH-OR | | | | | |
| $(CH_2)_m$ | c.) 3.85 | c.) 3.78 | c.) Multiplett | | |
| C(w-i) CHOR' | | | | | |
| $CH_3$ | 1.16 | 1.11 | Dublett J = 6 Hz | | |

a.)

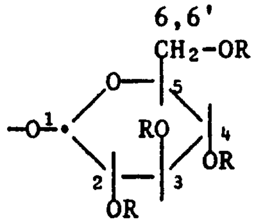

I (R = H)
Ia (R = $CH_3CO$)

b.) Die Signale der fünf Acetylgruppen erscheinen als Singuletts bei 2.04, 2.01, 2.00 (2x) und 1.95 ppm.
c.) Die Signale der $(CH_2)_m$- und $(CH_2)_n$-Gruppen absorbieren als Multipletts bei 1.60 und 1.45-1.25 ppm für 1 sowie 1.65-1.50 und 1.45-1.25 ppm für 1a.

## Patentansprüche

1. Die Verbindung der Formel

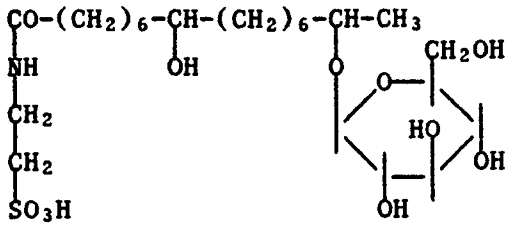

2. Die Verbindung erhältlich aus Exkrementen der Kirschenfliege Rhagoletis cerasi durch mehrfache chromatographische Reinigung.

3. Verwendung der Verbindung gemäss Anspruch 1 und 2 zum Schützen von Kirschen vor dem Befall durch Kirschenfliegen.

4. Verfahren zur Gewinnung der Verbindung gemäss Anspruch 1 und 2, dadurch gekennzeichnet, dass die Exkremente der Kirschenfliege Rhagoletis cerasi in Methanol aufgenommen und mehrfach mittels Säulen- und Dünnschicht-Chromatographie gereinigt werden.

## Claims

1. The compound of formula

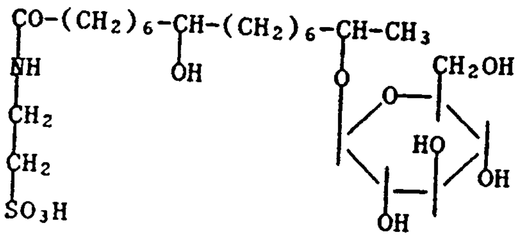

2. The compound obtainable from excrement of the cherry maggot Rhagoletis cerasi by repeated chromatographic purification.

3. Use of the compound as claimed in claims 1 and 2 for protecting cherries from attack by cherry maggots.

4. A process for obtaining the compound as claimed in claims 1 and 2, which comprises taking up the excrement of the cherry maggot Rhagoletis cerasi in methanol and effecting purification by column and thin-layer chromatography.

## Revendications

1. Le composé de formule

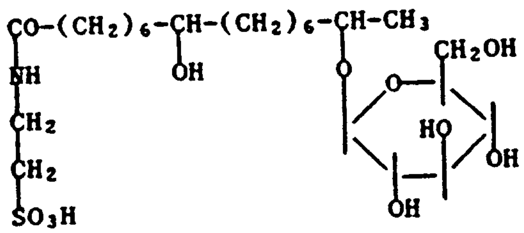

2. Le composé obtenu à partir d'excréments de la mouche de la cerise Rhagoletis cerasi par purifications chromatographiques répétées.

3. Utilisation du composé selon revendications 1 et 2 pour la protection des cerises contre l'attaque par la mouche de la cerise.

4. Procédé pour isoler le composé selon revendications 1 et 2, caractérisé en ce que l'on reprend dans le méthanol les excréments de la mouche de la cerise Rhagoletis cerasi et on soumet à purifications répétées par chromatographie sur colonne et chromatographie sur couche mince.